(19) Europäisches Patentamt / European Patent Office / Office européen des brevets

(11) **EP 4 088 815 B1**

(12) **EUROPEAN PATENT SPECIFICATION**

(45) Date of publication and mention
of the grant of the patent:
**05.06.2024 Bulletin 2024/23**

(21) Application number: **22172810.8**

(22) Date of filing: **11.05.2022**

(51) International Patent Classification (IPC):
**G01N 15/00** (2024.01)     **G01N 15/14** (2024.01)
**B01L 3/00** (2006.01)

(52) Cooperative Patent Classification (CPC):
**B01L 3/5085; G01N 15/1459;** B01L 2200/0689;
B01L 2200/12; B01L 2300/0803; B01L 2300/0806;
G01N 2015/0038; G01N 2015/1486

(54) **PRODUCTION METHOD FOR REACTION UNIT, PRODUCTION KIT FOR REACTION UNIT, AND MEASUREMENT METHOD FOR SUBSTANCE TO BE DETECTED**

HERSTELLUNGSVERFAHREN FÜR EINE REAKTIONSEINHEIT, HERSTELLUNGSKITS FÜR DIE REAKTIONSEINHEIT UND MESSVERFAHREN FÜR EINE NACHZUWEISENDE SUBSTANZ

PROCEDE DE PRODUCTION D'UNE UNITE DE REACTION, KIT DE PRODUCTION DE L'UNITE DE REACTION ET PROCEDE DE MESURE D'UNE SUBSTANCE A DETECTER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2021 JP 2021082279**

(43) Date of publication of application:
**16.11.2022 Bulletin 2022/46**

(73) Proprietor: **JVCKenwood Corporation
Yokohama-shi, Kanagawa 2210022 (JP)**

(72) Inventor: **OHSHIMA, Katsunori
Yokohama-shi, 221-0022 (JP)**

(74) Representative: **Schmidbauer, Andreas Konrad
Wagner & Geyer Partnerschaft mbB
Patent- und Rechtsanwälte
Gewürzmühlstrasse 5
80538 München (DE)**

(56) References cited:
**US-A1- 2008 063 572     US-A1- 2016 089 649
US-A1- 2020 217 864**

## Description

BACKGROUND OF THE INVENTION

Field of the Invention

The present invention relates to a method of producing a reaction unit, a kit for producing a reaction unit, and a method of measuring a detection target substance.

Priority is claimed on Japanese Patent Application No. 2021-082279, filed May 14, 2021.

Description of Related Art

As an immunochemical analysis method typified by an antigen-antibody reaction, a method of labeling a detection target substance with nanoparticles or the like (labeled particles) to which an antibody or the like that specifically binds to the detection target substance is fixed is known (for example, Japanese Unexamined Patent Application, First Publication No. 2017-207289). In this method, a reaction well is provided on an optical disc substrate to which an antibody is fixed, and an antigen-antibody reaction in a solution is performed in the well. Next, nanoparticles to which an antibody is fixed are bound to the detection target substance captured on the optical disc substrate, and a technique for optical disc reproduction is applied to measure the nanoparticles captured on the optical disc substrate.

In the above method, water-soluble reagents are added dropwise to the reaction well, and an antigen-antibody reaction in a solution is performed. Therefore, the contact parts between the reaction well and the optical disc substrate need to be in close contact with each other so that an aqueous solution does not leak. In the related art, a rubber packing made of polydimethylsiloxane (PDMS) is used for a contact part between the reaction well and the optical disc substrate, and thereby liquid leakage is prevented.

US 2020 217864 A discloses a dispensing unit which includes a disc, a cartridge, and a dispensing holder. The disc has a disc shape and includes a track region provided with recesses and projections alternately arranged in a radial direction. The cartridge includes a penetration hole, and a well is formed by the penetration hole and the track region in a state in which the cartridge is attached to the disc. The dispensing holder includes a holding part to be inserted into the penetration hole, and a guide hole penetrating the holding part. The guide hole has a truncated cone shape in which a first opening diameter on the holding part side is smaller than a second opening diameter on a side opposite to the holding part, and a center line of the guide hole is located on a line passing through the center of the disc and the center of the well.

US 2016 089649 A discloses a method for holding samples for analysis and an apparatus thereof which includes a testing plate with a pair of opposing surfaces and a plurality of holes. Each of the holes extends from one of the opposing surfaces to the other one of the opposing surfaces. The holes are arranged in groups, where each group has at least two rows and two columns of holes. The groups are arranged in sets, where each set has at least two rows and two columns of groups. To analyze samples, at least one of the opposing surfaces of the testing plate is immersed in a solution to be analyzed. A portion of the solution enters openings for each of the holes in the immersed opposing surface. Once the holes are filled with solution, the testing plate is removed and is held above a supporting surface. Surface tension holds the solution in each of the holes. The solution in one or more of the holes is then analyzed and the solution in one of these holes is identified for further study. The location of the identified solution is marked based upon its location within a particular set and group of holes.

US 2008 063572 A discloses a holding device for studying cells which includes at least one cavity adapted to receive cells in a medium consisting essentially of water, the cavity having a substrate and a generally inert wall. The substrate includes a surface for receiving the medium. The surface includes a multiplicity of pico liter wells. The substrate is substantially translucent and has a refractive index equal to the refractive index of the medium.

SUMMARY OF THE INVENTION

When the rubber packing made of PDMS is designed and produced once by molding or the like, it is necessary to design and reproduce it once again when the size or the like is changed. Therefore, changing the size or the like requires a long production period and additional cost. It is desirable to prevent liquid leakage from the well with a simple method.

In accordance with the present invention, a method and a kit set forth in the appended claims is provided.

BRIEF DESCRIPTION OF THE DRAWINGS

FIG. 1 is a perspective view of an example of a polypropylene substrate.
FIG. 2A is a top view of a polypropylene substrate 10 shown in FIG. 1.
FIG. 2B is a cross-sectional view of the polypropylene substrate 10 shown in FIG. 2A along the line B-B.
FIG. 3 is a perspective view of an example of a cycloolefin polymer (COP)/cycloolefin copolymer (COC) substrate.
FIG. 4A is a top view of the COP/COC substrate 20 shown in FIG. 3.
FIG. 4B is a schematic view illustrating a structure of a track region 21 of the COP/COC substrate 20 shown in FIG. 4A.

FIG. 5 is a schematic view illustrating one process of a method of producing a reaction unit according to one embodiment.

FIG. 6A is a schematic view illustrating one process of the method of producing a reaction unit according to one embodiment.

FIG. 6B is a schematic view illustrating one process of the method of producing a reaction unit according to one embodiment.

FIG. 7A is a schematic view showing an example of a reaction unit produced by the method of producing a reaction unit according to one embodiment.

FIG. 7B is a cross-sectional view of a reaction unit 101 shown in FIG. 7A along the line B-B.

FIG. 8A is a schematic view illustrating one process of the method of producing a reaction unit according to one embodiment.

FIG. 8B is a schematic view illustrating one process of the method of producing a reaction unit according to one embodiment.

FIG. 8C is a schematic view illustrating one process of the method of producing a reaction unit according to one embodiment.

FIG. 9A is a schematic view showing an example of a reaction unit produced by the method of producing a reaction unit according to one embodiment.

FIG. 9B is a cross-sectional view of a reaction unit 102 shown in FIG. 9A along the line B-B.

FIG. 10 is a schematic view illustrating one process of a method of measuring a detection target substance using the reaction unit according to one embodiment.

FIG. 11A is a schematic view illustrating one process of the method of measuring a detection target substance using the reaction unit according to one embodiment.

FIG. 11B is a schematic view illustrating one process of the method of measuring a detection target substance using the reaction unit according to one embodiment.

DETAILED DESCRIPTION OF THE INVENTION

Hereinafter, embodiments of the present invention will be described in detail with reference to the drawings in some cases. In the drawings, the same or corresponding components will be denoted with the same or corresponding reference numerals, and redundant descriptions thereof will be omitted. The dimensional ratios in the drawings may be exaggerated for explanation, and do not necessarily match actual dimensional ratios.

"Antibody" indicates an immunoglobulin having an antigen-binding activity. The antibody does not necessarily have to be an intact antibody as long as it has an antigen-binding activity, and may be an antigen-binding fragment. The term "antibody" used in this specification includes an antigen-binding fragment. An "antigen-binding fragment" is a polypeptide containing a part of an antibody, and is a polypeptide which maintains an antigen-binding activity of the original antibody. The antigen-binding fragment preferably includes all six complementarity determining regions (CDRs) of the original antibody. That is, it preferably includes all of heavy chain variable regions CDR1, CDR2, and CDR3 and light chain variable regions CDR1, CDR2, and CDR3. Examples of antigen-binding fragments include Fab, Fab', $F(ab')_2$, a variable region fragment (Fv), a disulfide bond Fv, a single chain Fv(scFv), and $sc(Fv)_2$.

The antibody may be derived from any organism. Examples of organisms from which the antibody is derived include mammals (humans, mice, rats, rabbits, horses, cattle, pigs, monkeys, dogs, etc.), birds (chickens and ostriches), but the present invention is not limited thereto.

The antibody may be in any class or subclass of immunoglobulin. The antibody may be a monoclonal antibody or a polyclonal antibody, and is preferably a monoclonal antibody.

The antibody can be produced by a known method such as an immunization method, a hybridoma method, and a phage display method.

"Having a specific binding property" means having a high binding property with respect to a target substance and having almost no binding property with respect to other substances.

[Method of producing reaction unit]

In one embodiment, the present disclosure provides a method of producing a reaction unit. A reaction unit obtained by the production method of the present embodiment is for performing an antigen-antibody reaction for capturing a detection target substance. After the antigen-antibody reaction, a detection target substance is measured using a counting device including an optical pickup (ExoCounter (registered trademark), etc.).

<First embodiment>

In one embodiment, a method of producing a reaction unit includes (a1) a process of preparing a first substrate which is a substrate having a first surface and a second surface and of which at least the first surface is composed of polypropylene, and which has one or more through-holes that penetrate from the first surface to the second surface, and a second substrate which is a substrate having a first surface and a second surface and of which at least the first surface is composed of at least one selected from the group consisting of cycloolefin polymers and cycloolefin copolymers, and which has a track region in which concave parts and convex parts are alternately formed on the first surface, (b1) a process of applying a photocurable composition around an opening of the through-hole on the first surface of the first substrate, (c1) a process of emitting light to the photocurable composition applied around the opening of the through-hole to form a cured resin layer in which the photocurable composition is cured, and (d1) a process of forming a well having the track region as a bottom surface and the through-hole as a side surface, which is a well formed by bringing the cured resin layer formed on the first surface of the first substrate into close contact with the first surface of the second substrate after the process (c1).

(Process (a 1)

In the process (a1), a first substrate which is a substrate having a first surface and a second surface and of which at least the first surface is composed of polypropylene, and which has one or more through-holes that penetrate from the first surface to the second surface, and a second substrate which is a substrate having a first surface and a second surface and of which at least the first surface is composed of at least one selected from the group consisting of cycloolefin polymers and cycloolefin copolymers, and which has a track region in which concave parts and convex parts are alternately formed on the first surface are prepared.

<<First substrate (polypropylene substrate)>>

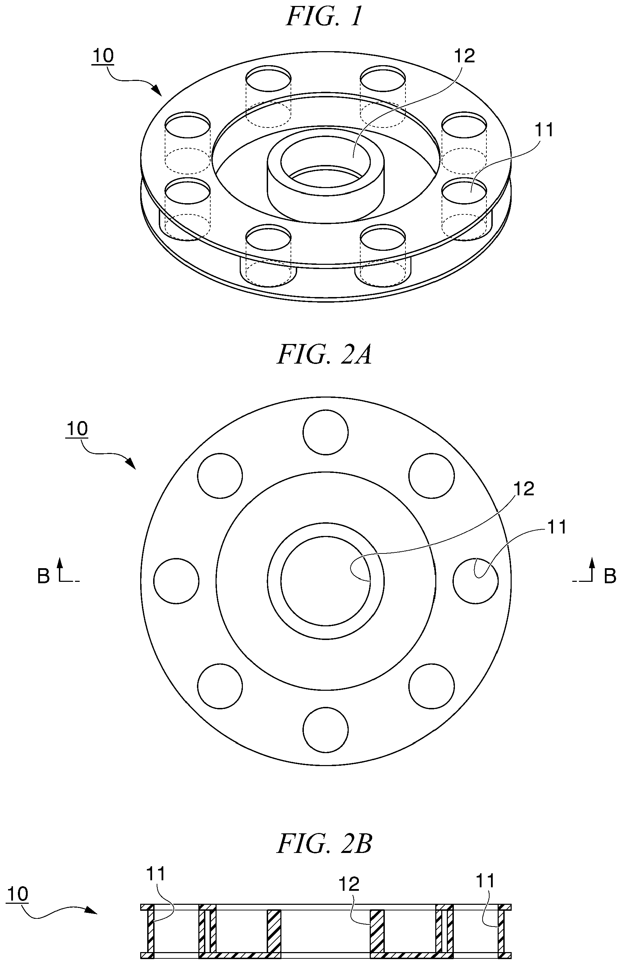

FIG. 1 is a perspective view showing an example of a first substrate (hereinafter also referred to as a "polypropylene substrate") used for producing a reaction unit. FIG. 2A is a top view of a polypropylene substrate 10 shown in FIG. 1. FIG. 2B is a cross-sectional view of the polypropylene substrate 10 shown in FIG. 2A along the line B-B.
The polypropylene substrate 10 has a positioning hole 12 and one or more through-holes 11.
The polypropylene substrate 10 has a disk shape, and has the positioning hole 12 in the center. Here, it is described that the positioning hole 12 is provided in the center, but any structure may be used as long as it can be positioned. For example, a projection structure may be used instead of the positioning hole 12. The polypropylene substrate 10 has a first surface and a second surface. The first surface is the lower surface in FIG. 1, FIG. 2A and FIG. 2B. The second surface is the upper surface in FIG. 1, FIG. 2A and FIG. 2B.
At least the first surface of the polypropylene substrate 10 is composed of polypropylene. The entire polypropylene substrate 10 may be composed of polypropylene, and at least one surface (a surface corresponding to the first surface) of a substrate composed of another synthetic resin (for example, polycarbonate, polystyrene, etc.) may be coated with polypropylene.
The size of the polypropylene substrate 10 can be appropriately selected according to the size of a COP/COC substrate 20 to be described below. In the polypropylene substrate 10, for example, the diameter of the outermost circle can be about 7 to 15 cm.

The thickness of the polypropylene substrate 10 is not particularly limited. The thickness of the polypropylene substrate 10 can be, for example, 5 to 15 mm.
The polypropylene substrate 10 may be hollow in order to reduce the weight and cost.
The through-hole 11 is formed so that it penetrates from the first surface to the second surface of the polypropylene substrate 10. The through-hole 11 has a cylindrical shape. Eight through-holes 11 are formed at equal intervals in the circumferential direction of the polypropylene substrate 10. The number and arrangement of through-holes 11 are not limited thereto, and any number and arrangement can be used. The size of the through-hole 11 is not particularly limited. For example, the through-hole 11 can have a cylindrical shape having a diameter of 5 to 15 mm.

<<Second substrate (COP/COC substrate)>>

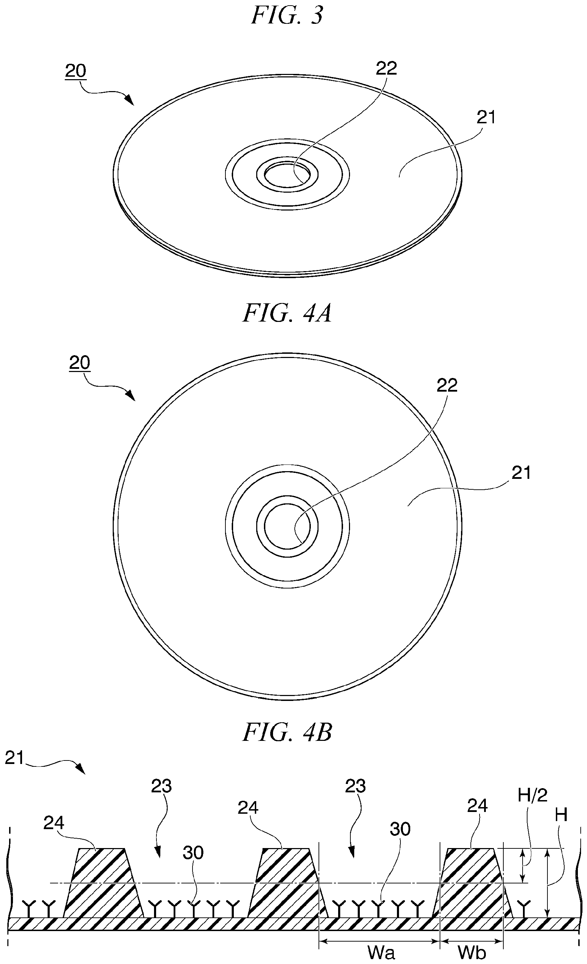

FIG. 3 is a perspective view showing an example of a second substrate (hereinafter also referred to as a "COP/COC substrate") used for producing a reaction unit. FIG. 4A is a top view of the COP/COC substrate 20 shown in FIG. 3. The COP/COC substrate 20 includes a track region 21 and a positioning hole 22. Here, it is described that the positioning hole 22 is provided in the center, but any structure may be used as long as it can be positioned. For example, a projection structure may be used instead of the positioning hole 22.
The COP/COC substrate 20 has a disk shape, and has the positioning hole 22 in the center. The COP/COC substrate 20 has a first surface and a second surface. The first surface is the upper surface in FIG. 3 and FIG. 4A. The second surface is the lower surface in FIG. 3 and FIG. 4A.
In the COP/COC substrate 20, at least the first surface is composed of at least one selected from the group consisting of cycloolefin polymers and cycloolefin copolymers. The entire COP/COC substrate 20 may be composed of at least one selected from the group consisting of cycloolefin polymers and cycloolefin copolymers, and at least one surface (a surface corresponding to the first surface) of a substrate composed of another synthetic resin (for example, polycarbonate, etc.) may be coated with at least one selected from the group consisting of cycloolefin polymers and cycloolefin copolymers. The first surface of the COP/COC substrate may be composed of a cycloolefin polymer, may be composed of a cycloolefin copolymer, or may be composed of a mixture of a cycloolefin polymer and a cycloolefin copolymer.
The COP/COC substrate 20 can have the same size as an optical disc such as a Blu-ray Disc (BD), a DVD, or a Compact Disc (CD). The COP/COC substrate 20 can have, for example, a diameter of about 7 to 15 cm.
FIG. 4B is a schematic view illustrating a structure of the track region 21. In the track region 21, concave parts 23 and convex parts 24 are alternately arranged in the radial direction of the COP/COC substrate 20. The concave parts 23 and the convex parts 24 are formed in spiral shapes from the inner peripheral part to the outer peripheral part of the track region 21. The concave parts 23 correspond to grooves of an optical disc. The convex parts 24 correspond to lands of an optical disc.

A capturing antibody 30 may be fixed to the track region 21. The capturing antibody 30 may be fixed only in the concave parts 23, or may be fixed in both the concave parts 23 and the convex parts 24. The capturing antibody 30 can be fixed to the track region 21 using a known method. For example, the track region 21 may be surface-treated so that proteins are physically adsorbed by a surface hydrophobic interaction, or may be surface-treated so that it has an amino group, a carboxyl group or the like. Alternatively, an avidin-biotin bond may be used.

(Process (b1))

In the process (b1), on the first surface of the polypropylene substrate, a photocurable composition is applied around an opening of a through-hole.

«Photocurable composition»

The photocurable composition is a composition containing a photopolymerizable compound. The photopolymerizable compound has a photopolymerizable group, and is polymerized and cured by light emission. The photopolymerizable group is not particularly limited, and examples thereof include a vinyl group, a methacryloyl group, an acryloyl group, and an epoxy group. Examples of resins formed by polymerization of a photopolymerizable compound include an acrylic resin and an epoxy resin, but the present invention is not limited thereto. An acrylic resin is preferable because it has elasticity and a strong effect of preventing liquid leakage between a polypropylene substrate and a COP/COC substrate. When a method of a second embodiment to be described below is used, bonding between a polypropylene substrate and a COP/COC substrate is performed with a photocurable composition, a series of reaction processes is completed, and then the process advances to a measurement process. In this case, the COP/COC substrate is peeled off from the polypropylene substrate at the interface with the photocurable composition. At that time, a resin having a property of being easily peeled off without leaving a residue on the surface of the COP/COC substrate is preferable.

Examples of photopolymerizable compounds forming an acrylic resin include monomers, oligomers, and polymers containing a methacryloyl group or an acryloyl group, and acrylic resins containing a photopolymerizable group. The term "(meth)acrylate" includes methacrylate and acrylate, and indicates methacrylate or acrylate.

Examples of monofunctional monomers containing a methacryloyl group or an acryloyl group include (meth)acrylates having an aliphatic polycyclic structure such as isobornyl (meth)acrylate, 1-adamantyl (meth)acrylate, 2-methyl-2-adamantyl (meth)acrylate, 2-ethyl-2-adamantyl (meth)acrylate, bornyl (meth)acrylate, and tricyclodecanyl (meth)acrylate; (meth)acrylates having an aliphatic monocyclic structure such as dicyclopentanyl (meth)acrylate, dicyclopentenyl(meth)acrylate, cyclohexyl (meth)acrylate, 4-butylcyclohexyl (meth)acrylate, and acryloyl morpholine; (meth)acrylates having a chain structure such as 2-hydroxyethyl (meth)acrylate, 2-hydroxypropyl (meth)acrylate, 2-hydroxybutyl (meth)acrylate, methyl (meth)acrylate, ethyl (meth)acrylate, propyl (meth)acrylate, isopropyl (meth)acrylate, butyl (meth)acrylate, amyl (meth)acrylate, isobutyl (meth)acrylate, t-butyl (meth)acrylate, pentyl (meth)acrylate, isoamyl (meth)acrylate, hexyl (meth)acrylate, heptyl (meth)acrylate, octyl (meth)acrylate, isooctyl (meth)acrylate, 2-ethylhexyl (meth)acrylate, nonyl (meth)acrylate, decyl (meth)acrylate, isodecyl (meth)acrylate, undecyl (meth)acrylate, dodecyl (meth)acrylate, lauryl (meth)acrylate, stearyl (meth)acrylate, and isostearyl (meth)acrylate; (meth)acrylates having an aromatic ring structure (hereinafter referred to as a "component (B2)") such as benzyl (meth)acrylate, phenoxyethyl (meth)acrylate, phenoxy-2-methylethyl (meth)acrylate, phenoxyethoxyethyl (meth)acrylate, 3-phenoxy-2-hydroxypropyl (meth)acrylate, 2-phenylphenoxyethyl (meth)acrylate, 4-phenylphenoxyethyl (meth)acrylate, 3-(2-phenylphenyl)-2-hydroxypropyl (meth)acrylate, (meth)acrylate of EO-modified p-cumylphenol, 2-bromophenoxyethyl (meth)acrylate, 2,4-dibromophenoxyethyl (meth)acrylate, 2,4,6-tribromophenoxyethyl (meth)acrylate, EO-modified phenoxy (meth)acrylate, PO-modified phenoxy (meth)acrylate, and polyoxyethylene nonylphenyl ether (meth)acrylate; and tetrahydrofurfuryl (meth)acrylate, butoxyethyl (meth)acrylate, ethoxydiethylene glycol (meth)acrylate, polyethylene glycol mono (meth)acrylate, polypropylene glycol mono (meth)acrylate, methoxyethylene glycol (meth)acrylate, ethoxyethyl (meth)acrylate, methoxypolyethylene glycol (meth)acrylate, and methoxypolypropylene glycol (meth)acrylate.

Examples of bifunctional monomers containing a methacryloyl group or an acryloyl group include trimethylolpropane di(meth)acrylate, ethylene glycol di(meth)acrylate, tetra ethylene glycol di(meth)acrylate, polyethylene glycol di(meth)acrylate, polypropylene glycol di(meth)acrylate, 1,4-butanediol di(meth)acrylate, 1,6-hexanediol di(meth)acrylate, neopentyl glycol di(meth)acrylate, and bis(hydroxymethyl) tricyclodecane di(meth)acrylate.

Examples of tri- or higher-functional monomers containing a methacryloyl group or an acryloyl group include trifunctional monomers such as ethoxylated (3) trimethylolpropane triacrylate, ethoxylated (3) trimethylolpropane trimethacrylate, ethoxylated (6) trimethylolpropane triacrylate, ethoxylated (9) trimethylolpropane triacrylate, ethoxylated (15) trimethylolpropane triacrylate, ethoxylated (20) trimethylolpropane triacrylate, pentaerythritol triacrylate, pentaerythritol trimethacrylate, propoxylated (3) glyceryl triacrylate, propoxylated (3) glyceryl triacrylate, propoxylated (5,5) glyceryl triacrylate, propoxylated (3) trimethylolpropane triacrylate, propoxylated (6) trimethylolpropane triacrylate, trimethylolpropane triacrylate, trimethylolpropane trimethacrylate, tris-(2-hydroxyethyl)-isocyanurate triacrylate, tris-(2-hydroxyethyl)-isocyanurate trimethacrylate, ε-caprolactone-modified tris-(2-acryloxyethyl)isocyanurate, EO-modified trimethylolpropane tri(meth)acrylate, PO-modified trimethylolpropane tri(meth)acrylate, and EO-, or PO-modified trimethylolpropane tri(meth)acrylate; tetrafunctional monomers such as ditrimethylolpropane tetraacrylate, ethoxylated (4) pentaerythritol tetraacrylate, and pentaerythritol tetra(meth)acrylate; and penta- or higher-functional monomers such as dipentaerythritol pentaacrylate and dipentaerythritol hexaacrylate.

Oligomers or polymers containing a methacryloyl group or an acryloyl group may be those obtained by polymerizing the above monomers.

The photopolymerizable compounds may be used alone or two or more thereof may be used in combination.

The photocurable composition may contain a photopolymerization initiator. The type of the photopolymerization initiator is not particularly limited, and known initiators can be used. Regarding the type of the photopolymerization initiator, for example, a radical polymerization initiator and a cationic polymerization initiator are exemplary examples.

Examples of photopolymerization initiators include carbonyl compounds such as aromatic ketones, acylphosphine oxide compounds, aromatic onium salt compounds, thio compounds, oxime ester compounds, alkylamine compounds, onium salts, sulfonium salts, phosphonium salts, and pyridinium salts. The photopolymerization initiator can be appropriately selected according to the type of the photopolymerizable compound.

The photopolymerization initiators may be used alone or two or more thereof may be used in combination.

The photocurable composition may contain a solvent in order to dissolve or disperse and mix an optional component such as a photopolymerizable compound and a photopolymerization initiator. The solvent is not particularly limited, and known solvents can be used. As the solvent, for example, organic solvents such as alcohols and ethers can be used. The solvents may be used alone or two or more thereof may be used in combination.

The photocurable composition may contain various additives such as an anti-deterioration agent, a mold releasing agent, a diluting agent, an antioxidant, a heat stabilizer, a flame retardant, a plasticizer, and a surfactant.

«Application of photocurable composition»

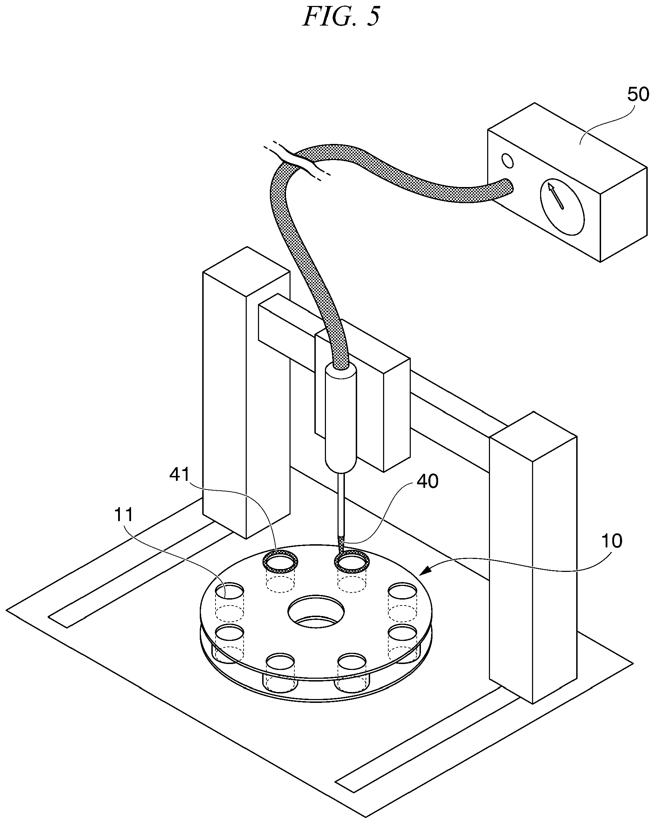

FIG. 5 is a schematic view illustrating the process (b1). On the first surface of the polypropylene substrate 10, a photocurable composition 40 is applied around the through-hole 11. Thereby, a photocurable composition layer 41 is formed around the through-hole 11. In the example in FIG. 5, a coating device 50 is used to apply the photocurable composition 40.

The photocurable composition layer 41 is seamlessly formed around the through-hole 11. The photocurable composition layer 41 is preferably formed along the end of the opening of the through-hole 11 according to the shape of the opening of the through-hole 11. In the example in FIG. 5, the photocurable composition layer 41 is formed in a ring shape along the circular shape of the opening of the cylindrical through-hole 11. The photocurable composition layer 41 is preferably formed to have a uniform width and thickness in order to prevent liquid leakage from between the polypropylene substrate 10 and the COP/COC substrate 20. The thickness of the photocurable composition layer 41 is not particularly limited, and may be, for example, about 0.5 to 5 mm. The width of the photocurable composition layer 41 is not particularly limited. The width of the photocurable composition layer 41 may be a width at which liquid leakage from between the polypropylene substrate 10 and the COP/COC substrate 20 can be prevented. The through-hole 11 of the polypropylene substrate 10 is formed based on the cylindrical shape. When the end of the cylindrical structure forming the through-hole 11 protrudes toward the first surface side of the polypropylene substrate 10, the outer wall thickness of the cylindrical structure, that is, the width from the inner diameter to the outer diameter of the cylindrical molded base material part, can be the width of the end of the cylindrical structure. In this case, the width of the photocurable composition layer 41 is preferably the same as the width of the end of the above cylindrical structure. That is, the width of the photocurable composition layer 41 is more preferably the same as the outer wall thickness at the tip of the cylindrical part constituting the through-hole 11 of the polypropylene substrate 10 on the side in contact with the COP/COC substrate 20. When the end of the cylindrical structure forming the through-hole 11 protrudes toward the first surface side of the polypropylene substrate 10, the width of the photocurable composition layer 41 is preferably the same as the width of the protruding end of the cylindrical structure. The width of the photocurable composition layer 41 may be, for example, about 0.5 to 10 mm.

(Process (c1))

In the process (c1), light is emitted to the photocurable composition applied around the opening of the through-hole to form a cured resin layer in which the photocurable composition is cured.

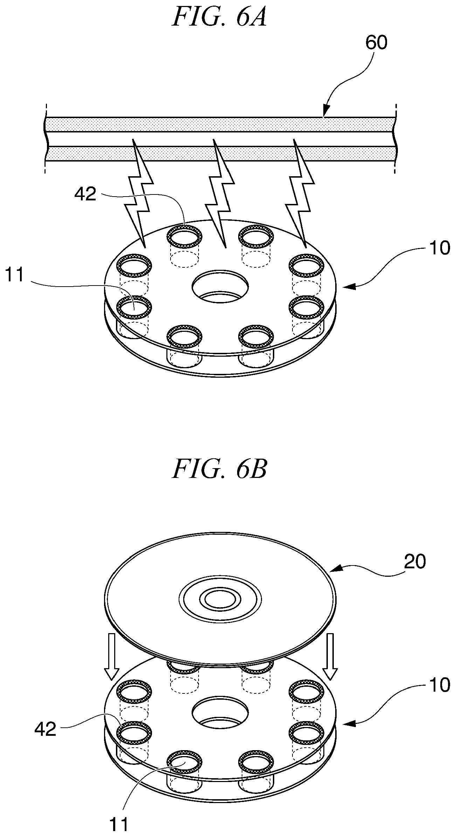

FIG. 6A is a schematic view illustrating the process (c1). Light is emitted to the photocurable composition layer 41 formed around the through-hole 11 of the polypropylene substrate 10 using a light emission device 60. Thereby, the photocurable composition layer 41 is cured to form a cured resin layer 42.

"Cured" means a state in which substantially all polymerizable groups in the photocurable composition have reacted. The "state in which substantially all polymerizable groups have reacted" means that most polymerizable groups in the photocurable composition have reacted, and even if there are unreacted polymerizable groups, the amount thereof is negligible. Even if light is emitted to the cured resin layer formed by curing the photocurable composition in the photocurable composition, additional curing does not proceed.

The type of light used for light emission is not particularly limited, and can be appropriately selected according to the type of the photopolymerizable compound contained in the photocurable composition. When the photopolymerizable compound is an acrylic compound, examples of light to be emitted include ultraviolet rays and visible light. The wavelength of light to be emitted may be, for example, 200 to 500 nm. The cumulative light amount required for curing may be, for example, 500 to 5,000 mJ/cm$^2$. Examples of light sources of ultraviolet rays or visible light include semiconductor-pumped solid-state lasers, carbon arc lamps, mercury lamps, metal halide lamps, xenon lamps, chemical lamps, and white LEDs.

(Process (d1))

In the process (d1), the COP/COC substrate and the polypropylene substrate are fixed so that the cured resin layer formed on the first surface of the polypropylene substrate is in close contact with the first surface of the COP/COC substrate, and a well having a bottom surface formed with the track region and a side surface formed with the through-hole is formed.

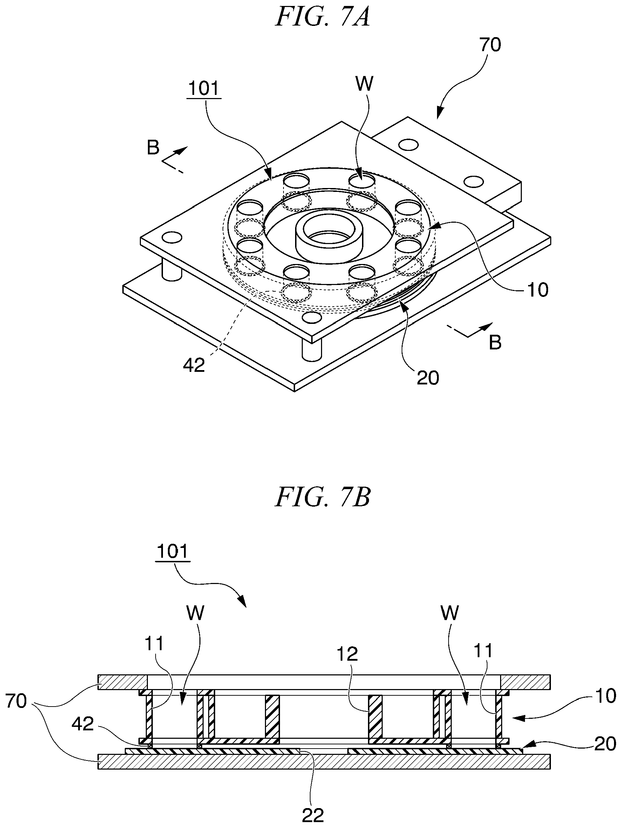

FIG. 6B and FIG. 7A are schematic views illustrating the process (d1). As shown in FIG. 6B, the COP/COC substrate 20 is placed on the first surface of the polypropylene substrate 10 so that the first surface of the polypropylene substrate 10 on which the cured resin layer 42 is formed and the first surface of the COP/COC substrate 20 face each other. In this state, fixing is performed using a fixing tool 70 or the like, and the first surface of the polypropylene substrate 10 is brought into close contact with the cured resin layer formed on the first surface of the COP/COC substrate 20 (refer to FIG. 7A). In this case, fixing is performed so that the opening of the through-hole 11 on the second surface of the polypropylene substrate 10 is not blocked by the fixing tool 70. Thereby, a well W having the track region 21 of the COP/COC substrate 20 as a bottom surface and the inner wall of the through-hole 11 of the polypropylene substrate 10 as a side surface is formed. That is, the cured resin layer 42 formed on the first surface of the polypropylene substrate 10 is in close contact with the first surface of the COP/COC substrate 20, and thus a well W having the track region 21 as a bottom surface and the through-hole 11 as a side surface is formed. The well W is used as a reaction chamber for an antigen-antibody reaction. The cured resin layer 42 functions as a packing and prevents liquid leakage from the gap between the track region 21 and the polypropylene substrate 10.

In this manner, it is possible to obtain a reaction unit 101 having a well W having a bottom surface composed of the track region 21 and a side surface composed of the through-hole 11.

FIG. 7B is a cross-sectional view of the reaction unit 101 in FIG. 7A along the line B-B. The cured resin layer 42 is formed around the through-hole 11 of the polypropylene substrate 10. The polypropylene substrate 10 and the COP/COC substrate 20 are in close contact with each other via the cured resin layer 42, and are fixed by the fixing tool 70 in this state. The cured resin layer 42 has elasticity, and functions as a packing that blocks between the polypropylene substrate 10 and the COP/COC substrate 20. Therefore, it is possible to effectively prevent liquid leakage from between the polypropylene substrate 10 and the COP/COC substrate 20.

<Second embodiment>

In one embodiment, a method of producing a reaction unit includes (a2) a process of preparing a first substrate which is a substrate having a first surface and a second surface and of which at least the first surface is composed of polypropylene, and which has one or more through-holes that penetrate from the first surface to the second surface, and a second substrate which is a substrate having a first surface and a second surface and of which at least the first surface is composed of a cycloolefin polymer, and which has a track region in which concave parts and convex parts are alternately formed on the first surface, (b2) a process of applying a photocurable composition around an opening of the through-hole on the first surface of the first substrate, (c2) a process of emitting light to the photocurable composition applied around the opening of the through-hole to form a semi-cured resin layer in which the photocurable composition is semi-cured, (d2) a process of forming a well having the track region as a bottom surface and the through-hole as a side surface, which is a well formed by bringing the semi-cured resin layer formed on the first surface of the first substrate into close contact with the first surface of the second substrate after the process (c2), and (e2) a process of curing the semi-cured resin layer and bonding the first surface of the first substrate and the first surface of the second substrate after the process (d2).

(Process (a2) and process (b2))

The process (a2) is the same as the process (a1). The process (b2) is the same as the process (b1).

(Process (c2))

In the process (c2), light is emitted to the photocurable composition applied around the opening of the through-hole to form a semi-cured resin layer in which the photocurable composition is semi-cured.

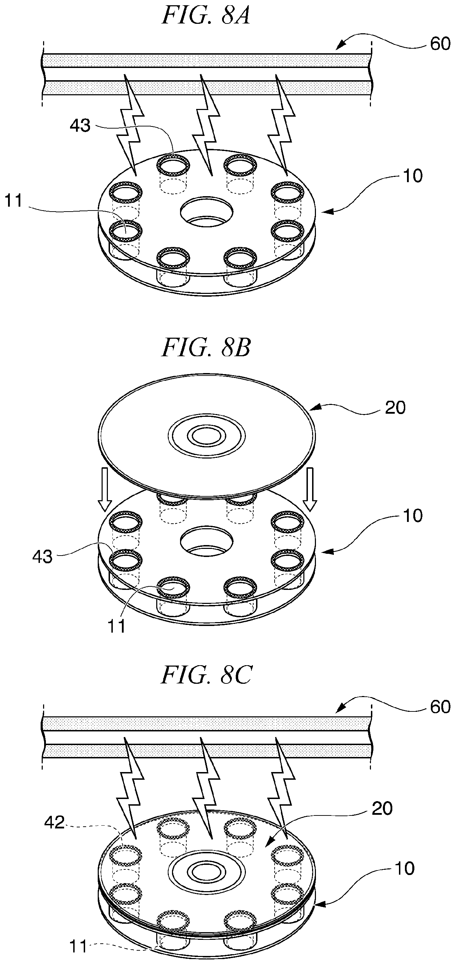

FIG. 8A is a schematic view illustrating the process (c2). Light is emitted to the photocurable composition layer 41 formed around the through-hole 11 of the polypropylene substrate 10 using the light emission device 60. In this case, light emission ends during the progress of the cure reaction of the photocurable composition layer 41. Thereby, the photocurable composition layer 41 is semi-cured to form a semi-cured resin layer 43.

"Semi-cured" means a state in which some polymerizable groups in the photocurable composition have reacted and some polymerizable groups have not reacted. When light is emitted to the semi-cured resin layer formed by semi-curing the photocurable composition in the photocurable composition, unreacted polymerizable groups react and curing proceeds.

The amount of light emission when the photocurable composition is semi-cured can be, for example, about 1/10 to 7/10 times the amount of light emission required for curing the photocurable composition.

(Process (d2))

In the process (d2), a well formed by bringing the semi-cured resin layer formed on the first surface of the polypropylene substrate into close contact with the first surface of the COP/COC substrate, which is a well having a bottom surface formed with the track region and a side surface formed with the through-hole, is formed.

FIG. 8B is a schematic view illustrating the process (d2). As shown in FIG. 8B, the COP/COC substrate 20 is placed on the first surface of the polypropylene substrate 10 so that the first surface of the polypropylene substrate 10 on which the semi-cured resin layer 43 is formed and the first surface of the COP/COC substrate 20 face each other. In this state, when both are pressed against each other, the semi-cured resin layer 43 formed on the first surface of the polypropylene substrate 10 can be brought into close contact with the first surface of the COP/COC substrate 20. Thereby, a well W having the track region 21 of the COP/COC substrate 20 as a bottom surface and the inner wall of the through-hole 11 of the polypropylene substrate 10 as a side surface is formed. That is, the semi-cured resin layer 43 formed on the first surface of the polypropylene substrate 10 is in close contact with the first surface of the COP/COC substrate 20, and thus a well W having the track region 21 as a bottom surface and the through-hole 11 as a side surface is formed.

(Process (e2))

In the process (e2), the semi-cured resin layer is cured to bond the first surface of the polypropylene substrate and the first surface of the COP/COC substrate.

FIG. 8C is a schematic view illustrating the process (e2). Light is emitted to the semi-cured resin layer 43 formed around the through-hole 11 of the polypropylene substrate 10 up to a cumulative light amount required for complete curing using the light emission device 60. Thereby, the semi-cured resin layer 43 is cured to form the cured resin layer 42 that bonds the first surface of the polypropylene substrate 10 and the first surface of the COP/COC substrate 20. The polypropylene substrate 10 and the COP/COC substrate 20 are bonded and fixed by the cured resin layer 42.

In this manner, it is possible to obtain a reaction unit 102 having a well W having a bottom surface composed of the track region 21 and a side surface composed of the through-hole 11.

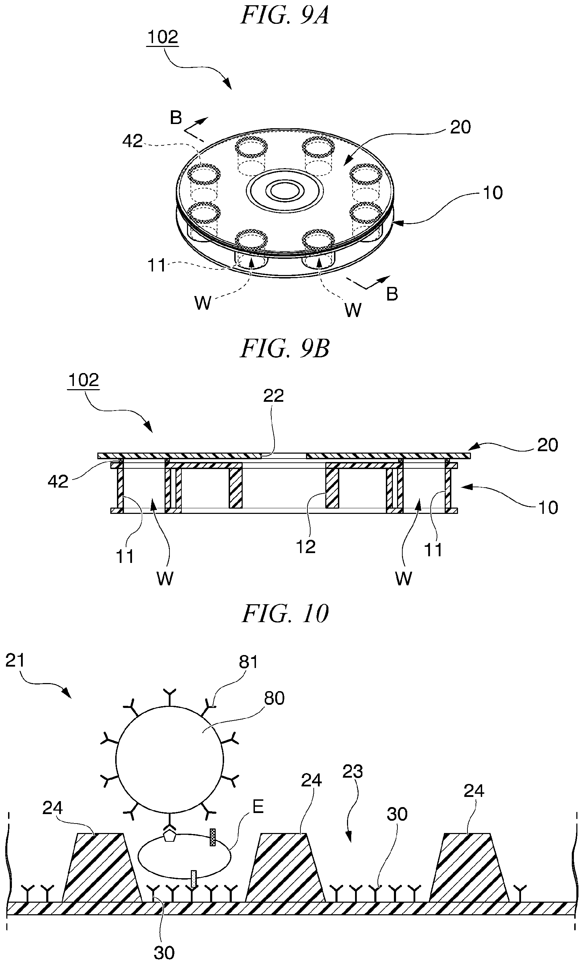

FIG. 9A is a perspective view showing the reaction unit 102 obtained above. FIG. 9B is a cross-sectional view of the reaction unit 102 in FIG. 9A along the line B-B. The cured resin layer 42 is formed around the through-hole 11 of the polypropylene substrate 10. The polypropylene substrate 10 and the COP/COC substrate 20 are bonded and fixed via the cured resin layer 42. The cured resin layer 42 bonds to the polypropylene substrate 10 and the COP/COC substrate 20, and blocks between them. Therefore, it is possible to effectively prevent liquid leakage from between the polypropylene substrate 10 and the COP/COC substrate 20.

According to production method of the present embodiment, the cured resin layer is formed around the through-hole in the polypropylene substrate, and thereby, liquid leakage from the bottom of the well is prevented. Since the photocurable composition is applied by a coating device or the like, it is not necessary to remake the packing even if the shape and size of the polypropylene substrate are changed. In addition, the photocurable composition that forms the cured resin layer is relatively inexpensive. Therefore, it is possible to reduce running cost.

[Kit for producing reaction unit]

In one embodiment, the present disclosure provides a kit for producing a reaction unit. The kit for producing a reaction unit of the present embodiment can be used to produce a reaction unit.

<Third embodiment>

In one embodiment, a kit for producing a reaction unit includes a first substrate which is a substrate having a first surface and a second surface and of which at least the first surface is composed of polypropylene, and which has one or more through-holes that penetrate from the first surface to the second surface, and in which a cured resin layer formed by photo-curing a photocurable composition is formed around an opening of the through-hole on the first surface, and a second substrate which is a substrate having a first surface and a second surface and of which at least the first surface is composed of at least one selected from the group consisting of cycloolefin polymers and cycloolefin copolymers, and which has a track region in which concave parts and convex parts are alternately formed on the first surface. When the first substrate and the second substrate are fixed so that the cured resin layer formed on the first surface of the first substrate is in close contact with the first surface of the second substrate, a well having the track region as a bottom surface and the through-hole as a side surface is formed.

(First substrate (polypropylene substrate))

The polypropylene substrate is the same as that prepared in the process (a1). When the polypropylene substrate prepared in the process (a1) is subjected to the process (b1) and the process (c1), it is possible to obtain a polypropylene substrate in which a cured resin layer obtained by photo-curing a photocurable composition is formed around an opening of a through-hole on the first surface. The "cured resin layer obtained by photo-curing a photocurable composition" is a cured resin layer in which a photocurable composition is cured by light emission.

(Second substrate (COP/COC substrate))

The COP/COC substrate is the same as that prepared in the process (a1). In the COP/COC substrate, the capturing antibody may or may not be fixed in the track region. When the capturing antibody is not fixed in the track region, a user can fix any antibody.
When the polypropylene substrate and the COP/COC substrate are subjected to the process (d1), they can be used as a reaction unit. That is, when the polypropylene substrate and the COP/COC substrate are fixed so that the cured resin layer formed on the first surface of the polypropylene substrate is in close contact with the first surface of the COP/COC substrate, a well having the track region as a bottom surface and the through-hole as a side surface is formed. The well can be used as a reaction well.

(Optional component)

The production kit of the present embodiment may include optional components in addition to the polypropylene substrate and the COP/COC substrate. Examples of optional components include a fixing tool for fixing a polypropylene substrate and a COP/COC substrate, nanoparticles used for an antigen-antibody reaction, various reagents such as a washing solution, a blocking solution, and a buffer solution, and instruction manuals.

<<Fixing tool>>

The fixing tool is used to fix the polypropylene substrate and the COP/COC substrate are fixed so that the first surface of polypropylene substrate is in close contact with the first surface of the COP/COC substrate, in order to form a well having a bottom surface formed with the track region and a side surface formed with the through-hole.
The fixing tool is not particularly limited as long as it can press and fix the polypropylene substrate and the COP/COC substrate. The fixing tool may include, for example, a bottom plate and a push plate, and may include a mechanism by which the push plate can move in the vertical direction. The bottom plate may be composed of a substrate having a larger size than the COP/COC substrate so that the COP/COC substrate can be placed thereon. In the bottom plate, a positioning member that can be inserted into positioning holes of the polypropylene substrate and the COP/COC substrate may be provided. In the bottom plate, a support column for guiding the push plate that moves in the vertical direction may be provided.
The push plate may be composed of a substrate having a larger size than the polypropylene substrate so that it can press the COP/COC substrate when the polypropylene substrate is laminated on the COP/COC substrate. A hole may be provided in the push plate so that the opening of the through-hole on the second surface of the polypropylene substrate is not blocked. The hole may be, for example, a hole having a size slightly smaller than the size of the polypropylene substrate so that the push plate can press the end of the polypropylene substrate.
The fixing tool may include a push plate fixing mechanism for fixing the push plate when the polypropylene substrate is pressed against the COP/COC substrate. For example, examples of push plate fixing mechanisms include screws.

<<Nanoparticles>>

Nanoparticles are used to measure a detection target substance captured with the capturing antibody fixed in the track region. Nanoparticles having a surface to which a detection antibody that specifically binds to a detection target substance is bound are used. The detection antibody may or may not be bound to the nanoparticles. When the detection antibody is not bound to the nanoparticles, the user can bind any detection antibody to the nanoparticles. In this case, the surface of the nanoparticles may be subjected to surface modification so that the detection antibody is easily bound. Examples of such surface modification include carboxy group modification.

"Nanoparticles" indicates particles having an average primary particle size on the order of nanometers (less than 1,000 nm). The average primary particle size of the nanoparticles can be appropriately selected according to the detection target substance. The average primary particle size of the nanoparticles may be, for example, 10 to 1,000 nm, 50 to 500 nm, 100 to 300 nm, or 150 to 200 nm.

The material of the nanoparticles is not particularly limited. Examples of materials of the nanoparticles include resins such as polystyrene and glycidyl methacrylate; metals such as iron, gold, and silver; metal oxides such as zirconia, titania, and iron oxide; and magnetic materials such as ferrite, but the present invention is not limited thereto.

When the detection target substance is an exosome, the relationship among the depth (height of the convex part 24) H of the concave part 23, the width Wa of the concave part 23, the width Wb of the convex part 24, the particle size Rc of nanoparticles, and the particle size Ra of the exosome will be described with reference to FIG. 4B. The width Wa and the width Wb are widths at the position of H/2 indicated by dotted lines.

The depth (height of the convex part 24) H of the concave part 23, the width Wa of the concave part 23, the width Wb of the convex part 24, the particle size Rc of nanoparticles, and the particle size Ra of the exosome preferably satisfy any of the following Formulae (1) to (6), and more preferably satisfy all of them.

$$Wb < Ra \cdots (1)$$

$$Ra < Wa < 4 \times Ra \cdots (2)$$

$$Wb < Rc < Wa < 2 \times Rc \cdots (3)$$

$$Ra < Rc \cdots (4)$$

$$(Ra + Rc)/8 < H \cdots (5)$$

$$(Ra + Rc)/6 < H \cdots (6)$$

When Formulae (1) to (6) are satisfied, the exosome easily enters the concave part 23, and nanoparticles easily enter the concave part 23. In addition, it is difficult for a plurality of nanoparticles to bind to the exosome captured in the concave part 23, and the quantitative relationship between the exosome and the nanoparticles can be brought closer to 1:1.

<Fourth embodiment>

In one embodiment, a kit for producing a reaction unit includes a first substrate which is a substrate having a first surface and a second surface and of which at least the first surface is composed of polypropylene, and has one or more through-holes that penetrate from the first surface to the second surface and in which a semi-cured resin layer obtained by photo-semi-curing a photocurable composition is formed around an opening of the through-hole on the first surface, and a second substrate which is a substrate having a first surface and a second surface and of which at least the first surface is composed of a cycloolefin polymer, and which has a track region in which concave parts and convex parts are alternately formed on the first surface.

(First substrate (polypropylene substrate))

The polypropylene substrate is the same as that prepared in the process (a2). When the polypropylene substrate prepared in the process (a2) are subjected to the process (b2) and the process (c2), it is possible to obtain a polypropylene substrate in which a semi-cured resin layer obtained by semi-curing a photocurable composition is formed around an opening of a through-hole on the first surface. The "cured resin layer obtained by photo-semi-curing a photocurable composition" is a semi-cured resin layer in which a photocurable composition is semi-cured by light emission.

(Second substrate (COP/COC substrate))

The COP/COC substrate is the same as that prepared in the process (a2). In the COP/COC substrate, the capturing antibody may or may not be fixed in the track region. When the capturing antibody is not fixed in the track region, the user can fix any antibody.

When the polypropylene substrate and the COP/COC substrate are subjected to the processes (d2) and (e2), they can be used as a reaction unit. That is, when the polypropylene substrate and the COP/COC substrate are bonded so that the semi-cured resin layer formed on the first surface of the polypropylene substrate is adhesively bonded to the first surface of the COP/COC substrate, a well having the track region as a bottom surface and the through-hole as a side surface is formed. The well can be used as a reaction well.

(Optional component)

The production kit of the present embodiment may include optional components in addition to the polypropylene substrate and the COP/COC substrate. Examples of optional components include the same ones as in the production kit of the third embodiment.

[Reaction unit]

<Fifth embodiment>

In one embodiment, the present disclosure provides a reaction unit. The reaction unit includes a first substrate which is a substrate having a first surface and a second surface and of which at least the first surface is composed of polypropylene and has one or more through-holes that penetrate from the first surface to the second surface, and in which a cured resin layer obtained by curing a photocurable composition is formed around an opening of the through-hole on the first surface, and a second substrate which is a substrate having a first surface and a second surface and of which at least the first surface is composed of a cycloolefin polymer, and which has a track region in which concave parts and convex parts are alternately formed on the first surface. The first substrate and the second substrate are fixed so that the cured resin layer formed on the first surface of the first substrate is in close contact with the first surface of the second substrate, and a well having the track region as a bottom surface and the through-hole as a side surface is formed.

The reaction unit of the present embodiment can be produced by the method of producing a reaction of the first embodiment. The configuration of the reaction unit of the present embodiment is as described above. In the track region of the COP/COC substrate, the capturing antibody may or may not be fixed. When the capturing antibody is not fixed in the track region, the user can fix any antibody.

<Sixth embodiment>

In one embodiment, the present disclosure provides a reaction unit. The reaction unit includes a first substrate which is a substrate having a first surface and a second surface and of which at least the first surface is composed of polypropylene, and which has one or more through-holes that penetrate from the first surface to the second surface, and in which a cured resin layer formed by photo-curing a photocurable composition is formed around an opening of the through-hole on the first surface, and a second substrate which is a substrate having a first surface and a second surface and of which at least the first surface is composed of a cycloolefin polymer, and which has a track region in which concave parts and convex parts are alternately formed on the first surface. The first surface of the first substrate and the first surface of the second substrate are bonded by the cured resin layer, and a well having the track region as a bottom surface and the through-hole as a side surface is formed.

The reaction unit of the present embodiment can be produced by the method of producing a reaction unit of the second embodiment. The configuration of the reaction unit of the present embodiment is as described above. In the track region of the COP/COC substrate, the capturing antibody may or may not be fixed. When the capturing antibody is not fixed in the track region, the user can fix any antibody.

[Measurement method]

In one embodiment, the present invention provides a method of measuring a detection target substance. The measurement method of the present embodiment includes (i) a process of adding a sample containing a detection target substance having a specific binding property with respect to a capturing antibody to a well of the reaction unit of the embodiment in which the capturing antibody is fixed in a track region, and performing a binding reaction between the capturing antibody and the detection target substance, (ii) a process of adding nanoparticles to which a detection antibody having a specific binding property with respect to the detection target substance is fixed to the well, bringing the detection target substance bound to the capturing antibody into contact with a nanoparticle to which a detection antibody having a specific binding property with respect to the detection target substance is fixed, and performing a binding reaction between the detection target substance and the detection antibody, (iii) a process of separating the first substrate and the second substrate after the process (ii), and (iv) a process of counting the nanoparticle captured on the separated second substrate.

The detection target substance is a substance to be measured by the measurement method of the present embodiment. The detection target substance is not particularly limited as long as it is a substance that causes an antigen-antibody reaction. Examples of detection target substances include proteins, peptides, sugar chains, and lipids as well as cells, viruses and extracellular vesicles expressing the compound, but the present invention is not limited thereto. Extracellular vesicles are vesicles released from cells. The size of extracellular vesicles is about 30 nm to 1 $\mu$m in diameter. Examples of extracellular vesicles include exosomes, apoptotic bodies, and microvesicles.

(Process (i))

In the process (i), a sample containing a detection target substance having a specific binding property with respect to a capturing antibody is added to a well of the reaction unit of the embodiment in which the capturing antibody is fixed in a track region, and a binding reaction between the capturing antibody and the detection target substance is performed.

The reaction unit may be either the reaction unit of the fifth embodiment or the reaction unit of the sixth embodiment. The reaction unit of the fifth embodiment can be produced by the method of producing a reaction unit of the first embodiment. The reaction unit of the sixth embodiment can be produced by the method of producing a reaction unit of the second embodiment. As the reaction unit, a unit in which a capturing antibody is fixed in the track region is used.

The "capturing antibody" is an antibody used for capturing a detection target substance on a COP/COC substrate. As the capturing antibody, an antibody having a specific binding property with respect to a detection target substance is used. When the detection target substance is an exosome, the capturing antibody may be an antibody having a specific binding property with respect to pan-exosome membrane proteins. Pan-exosome membrane proteins are proteins that are ubiquitous among exosomes. "Ubiquitous among exosomes" means that a wide variety of types of exosomes have them. Examples of pan-exosome membrane proteins include CD9, CD63, and CD81. Alternatively, the capturing antibody may be an antibody having a specific binding property with respect to membrane proteins that only some exosomes have. For example, cancer cells or exosomes released from cancer cells may have cancer antigens as membrane proteins. Therefore, when the detection target substance is a cancer cell-derived exosome, an antibody having a specific binding property with respect to a cancer antigen may be used. Examples of cancer antigens include Her2, CD147, MUC1, CEA, mesothelin, EGFR, EGFRvIII, MAGE, NY-ESO-1, PSMA, PSA, CD19, VEGFR1, and VEGFR2, but the present invention is not limited thereto.

The sample is a sample to be measured for a detection target substance. Any sample can be used. Examples of samples include body fluid samples (blood, serum, plasma, saliva, urine, tears, sweat, milk, nasal mucus, semen, pleural effusion, gastrointestinal secretions, cerebrospinal fluids, interstitial fluids, lymph fluids, etc.), and cell culture supernatants, but the present invention is not limited thereto. The sample may be a sample obtained by diluting the above body fluid sample, culture supernatant or the like. A buffer solution such as PBS can be used to dilute the sample.

The sample is added to the well of the reaction unit. When a binding reaction is performed under appropriate conditions, the detection target substance in the sample binds to the capturing antibody fixed in the track region of the COP/COC substrate.

The binding reaction can be performed by incubation for a predetermined time when the sample is added to the well. The incubation time may be a time sufficient for the detection target substance in the sample to bind to the capturing antibody. The incubation time may be, for example, 30 minutes or longer, 1 to 10 hours, 2 to 6 hours, 2 to 4 hours, or 2 to 3 hours. The incubation temperature may be, for example, 10 to 40°C, 20 to 40°C, or 30 to 40°C. In order to improve efficiency of the reaction between the capturing antibody and the detection target substance, the reaction unit may be gently shaken during incubation.

After the binding reaction, appropriately, the well may be washed using a washing solution. When the well is washed, it is possible to remove a substance that is not bound to the capturing antibody. The washing solution is not particularly limited, and washing solutions that are generally used in an immunochemical detection method can be used without particular limitation. Examples of washing solutions include buffer solutions such as PBS, a Tris buffer solution, and a HEPES buffer solution; solutions obtained by adding a surfactant such as Tween20 to the buffer solution; and pure water, but the present invention is not limited thereto.

The well may be subjected to a blocking treatment before the sample is added to the well. The blocking treatment can be performed by adding the blocking solution to the well and incubation. The blocking solution is not particularly limited, and solutions that are generally used in an immunochemical detection method can be used without particular limitation. Examples of blocking solutions include buffer solutions containing about 1 to 5% of skim milk, casein, or bovine serum albumin (BSA). The buffer solution for the blocking solution is not particularly limited, and examples thereof include PBS, PBS-T, a Tris buffer solution, and a HEPES buffer solution.

The incubation temperature may be, for example, 10 to 40°C, 20 to 40°C, or 30 to 40°C. The incubation time may be 10 to 180 minutes, 20 to 120 minutes, 20 to 100 minutes, or 30 to 60 minutes. When a blocking treatment is performed on a reaction region 210, it is possible to reduce non-specific adsorption with respect to the well.

(Process (ii))

In the process (ii), nanoparticles to which a detection antibody having a specific binding property with respect to a detection target substance is fixed are added to the well, the detection target substance bound to the capturing antibody is brought into contact with a nanoparticle to which a detection antibody having a specific binding property with respect to a detection target substance is fixed, and thus the binding reaction between the detection target substance and the detection antibody is performed.

The "detection antibody" is an antibody used to label a detection target substance with nanoparticles. The detection antibody is fixed to nanoparticles. As the detection antibody, an antibody having a specific binding property with respect to a detection target substance is used.

For example, when the detection target substance is an exosome, the detection antibody may be an antibody having a specific binding property with respect to pan-exosome membrane proteins. Alternatively, the detection antibody may be an antibody having a specific binding property with respect to membrane proteins that only some exosomes have. When the detection target substance is an exosome, the detection antibody and the capturing antibody may be an antibody having a specific binding property with respect to the same pan-exosome membrane protein (for example, CD9). Alternatively, the detection antibody and the capturing antibody may be antibodies having a specific binding property with respect to different pan-exosome membrane proteins (for example, CD9 and CD63). Alternatively, the capturing antibody may be an antibody having a specific binding property with respect to pan-exosome membrane proteins (for example, CD9), and the detection antibody may be an antibody having a specific binding property with respect to membrane proteins (for example, cancer antigens such as Her2 and CD147) specifically expressed in exosomes to be detected (for example, cancer cell-specific exosome).

After the process (i), nanoparticles to which a detection antibody is fixed are added to the well. When a binding reaction is performed under appropriate conditions, the detection antibody fixed to the nanoparticles binds to the detection target substance captured with the capturing antibody.

The binding reaction can be performed by incubation for a predetermined time when the nanoparticles are added to the well. The incubation time may be a time sufficient for the detection antibody to bind to the detection target substance. The incubation time may be, for example, 30 minutes or longer, 1 to 10 hours, 2 to 6 hours, 2 to 4 hours, or 2 to 3 hours. The incubation temperature may be, for example, 10 to 40°C, 20 to 40°C, or 30 to 40°C. In order to improve efficiency of the reaction between the detection antibody and the detection target substance, the reaction unit may be gently shaken during incubation.

After the binding reaction, appropriately, the well may be washed using a washing solution. When the well is washed, it is possible to remove a substance that is not bound to the capturing antibody. The washing solution is not particularly limited, and washing solutions that are generally used in an immunochemical detection method can be used without particular limitation. Examples of washing solutions include buffer solutions such as PBS, a Tris buffer solution, and a HEPES buffer solution; solutions obtained by adding a surfactant such as Tween20 to the buffer solution; and pure water, but the present invention is not limited thereto.

FIG. 10 is a schematic view illustrating a state of the track region after the process (ii). An exosome E is captured with the capturing antibody 30 fixed to the concave part 23 in the track region 21. The exosome E expresses a membrane protein having a specific binding property with respect to the capturing antibody 30. When the membrane protein binds to the capturing antibody 30, the exosome E is captured in the concave part 23.

The exosome E also expresses a membrane protein having a specific binding property with respect to a detection antibody 81 fixed to a nanoparticle 80. When the detection antibody 81 binds to the membrane protein, the nanoparticle 80 is captured in the concave part 23 via the exosome E.

(Process (iii))

In the process (iii), the first substrate and the second substrate are separated.

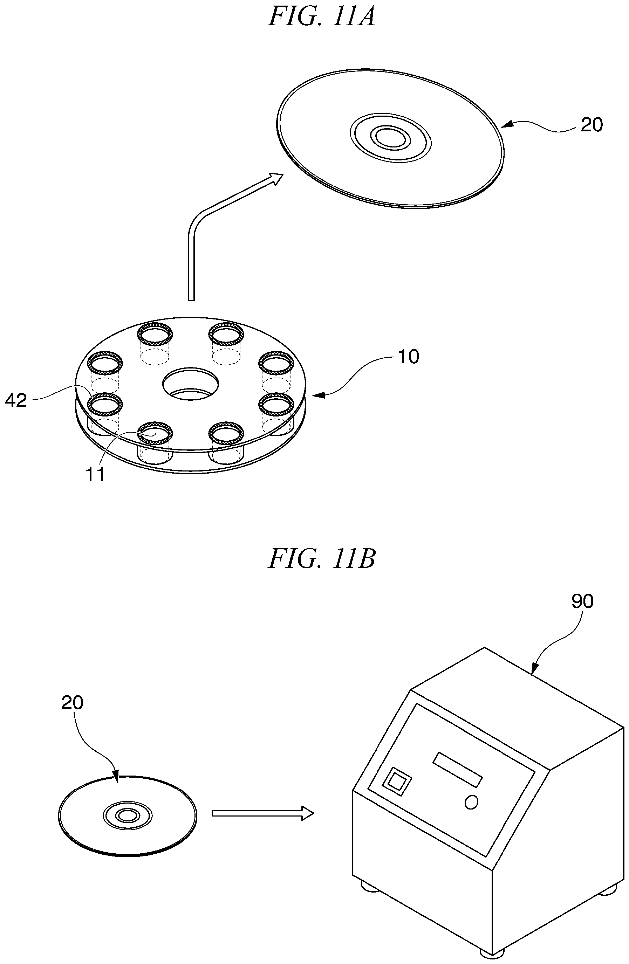

FIG. 11A is a schematic view illustrating the process (iii).
When the reaction unit is the reaction unit of the fifth embodiment, the polypropylene substrate 10 and the COP/COC substrate 20 can be separated by releasing fixing between the polypropylene substrate 10 and the COP/COC substrate 20.
When the reaction unit is the reaction unit of the sixth embodiment, the COP/COC substrate 20 is peeled off while holding the polypropylene substrate 10. Thereby, the polypropylene substrate 10 and the COP/COC substrate 20 can be separated. In the reaction unit of the sixth embodiment, the polypropylene substrate 10 and the COP/COC substrate 20 are bonded by the cured resin layer 42. The cured resin layer 42 formed by curing a photocurable resin has a higher bonding ability to polypropylene than the cycloolefin polymer. Therefore, when the COP/COC substrate 20 is peeled off from the polypropylene substrate 10, the cured resin layer 42 remains on the polypropylene substrate 10, and does not remain on the COP/COC substrate 20. Since no residue of the cured resin layer 42 remains, in the following process (iv), nanoparticles captured in the track region 21 of the COP/COC substrate 20 can be accurately counted.

(Process (iv))

In the process (iv), the nanoparticle captured in the separated COP/COC substrate are counted.
FIG. 11B is a schematic view illustrating the process (iv). In the process (iii), for the COP/COC substrate 20 separated from the polypropylene substrate 10, the number of nanoparticles captured in the track region 21 of the COP/COC substrate 20 is counted. The number of nanoparticles captured in the track region 21 reflects the amount of the detection target substance in the sample. Therefore, when nanoparticles captured in the track region 21 are counted, the detection substance in the sample can be indirectly measured.
Nanoparticles captured in the track region 21 of the COP/COC substrate 20 can be counted using a counting device 90. The counting device 90 is a counting device including an optical pickup (ExoCounter (registered trademark), etc.). The optical pickup emits a laser beam toward the track region 21. The laser beam is focused on the track region 21 by an objective lens. The wavelength of the laser beam is, for example, about 405 nm.
The optical pickup receives reflected light from the track region 21, detects a light receiving level of the reflected light, generates a light receiving level signal, and outputs the result to a control unit including a CPU and the like. In the control unit, a nanoparticle detection signal is extracted from the light receiving level signal output from the optical pickup. The track region 21 is driven at a certain linear velocity, and the laser beam is scanned along the concave part 23. The optical pickup extracts a nanoparticle detection signal along the concave part 23 and thus counts the nanoparticles captured in the track region 21.

Examples of a measurement device including such a mechanism include a counting device described in Japanese Unexamined Patent Application, First Publication No. 2017-207289.

According to the present invention, it is possible to provide a method of producing a reaction unit, a kit for producing a reaction unit, and a method of measuring a detection target substance using the reaction unit through which it is possible to prevent liquid leakage from a well by an inexpensive method.

Although preferable embodiments of the present invention have been described and illustrated above, it should be understood that these are examples of the present invention, and should not be considered limiting. Additions, omissions, substitutions, and other modifications can be performed without departing from the spirit and scope of the present invention. Therefore, the present invention is not considered to be limited by the above descriptions but only by the scope of appended claims.

The present disclosure includes matters that contribute to realization of "health and welfare for all people" through SDGs and that contribute to creation of value through healthcare products and services.

EXPLANATION OF REFERENCES

10 Polypropylene substrate (first substrate)
11 Through-hole
12 Positioning hole
20 COP/COC substrate (second substrate)
21 Track region
22 Positioning hole
23 Concave part
24 Convex part
30 Capturing antibody
40 Photocurable composition
41 Photocurable composition layer
42 Cured resin layer
43 Semi-cured resin layer
50 Coating device
60 Light emission device
70 Fixing tool
80 Nanoparticle
81 Detection antibody

**Claims**

1. A method of producing a reaction unit, comprising:

(a1) preparing a first substrate (10) which is a substrate having a first surface and a second surface and of which at least the first surface is composed of polypropylene, and which has one or more through-holes (11) that penetrate from the first surface to the second surface, and a second substrate (20) which is a substrate having a first surface and a second surface and of which at least the first surface is composed of at least one selected from the group consisting of cycloolefin polymers and cycloolefin copolymers, and which has a track region (21) in which concave parts and convex parts are alternately formed on the first surface;

(b1) applying a photocurable composition (41) around an opening of the through-hole (11) on the first surface of the first substrate (10);

(c1) emitting light to the photocurable composition (41) applied around the opening of the through-hole (11) to form a cured acrylic resin layer (42) in which the photocurable composition is cured, the cured acrylic resin layer (42) having elasticity; and

(d1) forming a well (W) having the track region (21) as a bottom surface and the through-hole (11) as a side surface by fixing the first substrate (10) and the second substrate (20) using a fixing tool (70), which is a well formed by bringing the cured acrylic resin layer (42) formed on the first surface of the first substrate (10) into close contact with the first surface of the second substrate (20) after (c1).

2. A method of producing a reaction unit, comprising:

(a2) preparing a first substrate (10) which is a substrate having a first surface and a second surface and of which at least the first surface is composed of polypropylene, and which has one or more through-holes (11) that penetrate from the first surface to the second surface, and a second substrate (20) which is a substrate having a first surface and a second surface and of which at least the first surface is composed of at least one selected from the group consisting of cycloolefin polymers and cycloolefin copolymers, and which has a track region (21) in which concave parts and convex parts are alternately formed on the first surface;

(b2) applying a photocurable composition (41) around an opening of the through-hole (11) on the first surface (10) of the first substrate;

(c2) emitting light to the photocurable composition (41) applied around the opening of the through-hole (11) to form a semi-cured acrylic resin layer (43) in which the photocurable composition is semi-cured, the semi-cured acrylic resin layer (43) is in a state in which some polymerizable groups in the photocurable composition (41) have reacted and some polymerizable groups have not reacted;

(d2) forming a well (W) having the track region (21) as a bottom surface and the through-hole (11) as a side surface, which is a well formed by bringing the semi-cured acrylic resin layer (43) formed on the first surface of the first substrate (10) into close contact with the first surface of the second substrate (20) after (c2); and

(e2) emitting light to the semi-cured acrylic resin layer (43) to form a cured acrylic resin layer (42) and thereby to bond the first surface of the first substrate (10) and the first surface of the second substrate (20) after (d2).

3.    A kit for producing a reaction unit, comprising:

a first substrate (10) which is a substrate having a first surface and a second surface and of which at least the first surface is composed of polypropylene, and which has one or more through-holes (11) that penetrate from the first surface to the second surface, and in which a cured acrylic resin layer (42) formed by photo-curing a photocurable composition is formed around an opening of the through-hole (11) on the first surface; and

a second substrate (20) which is a substrate having a first surface and a second surface and of which at least the first surface is composed of at least one selected from the group consisting of cycloolefin polymers and cycloolefin copolymers, and which has a track region (21) in which concave parts and convex parts are alternately formed on the first surface,

a fixing tool (70) configured to press and fix the first substrate (10) and the second substrate (20),

wherein a well (W) having the track region (21) as a bottom surface and the through-hole (11) as a side surface is formed when the first substrate (10) and the second substrate (20) are fixed so that the cured acrylic resin layer (42) formed on the first surface of the first substrate (10) is in close contact with the first surface of the second substrate (20) by the fixing tool (70).

4.    A kit for producing a reaction unit, comprising:

a first substrate (10) which is a substrate having a first surface and a second surface and of which at least the first surface is composed of polypropylene, and has one or more through-holes (11) that penetrate from the first surface to the second surface and in which a semi-cured acrylic resin layer (43) obtained by photo-semi-curing a photocurable composition is formed around an opening of the through-hole (11) on the first surface, the semi-cured acrylic resin layer (43) is in a state in which some polymerizable groups in the photocurable composition (41) have reacted and some polymerizable groups have not reacted; and

a second substrate (20) which is a substrate having a first surface and a second surface and of which at least the first surface is composed of at least one selected from the group consisting of cycloolefin polymers and cycloolefin copolymers, and which has a track region (21) in which concave parts and convex parts are alternately formed on the first surface,

wherein a well (W) having the track region (21) as a bottom surface and the through-hole (11) as a side surface is formed when the first substrate (10) and the second substrate (20) are bonded so that the semi-cured acrylic resin layer (43) formed on the first surface of the first substrate (10) is adhesively bonded to the first surface of the second substrate (20).

5.    A method of measuring a detection target substance, comprising:

(i) adding a sample containing a detection target substance having a specific binding property with respect to a capturing antibody (30) to a well (W) in which the capturing antibody (30) is fixed in a track region (21), which is the well (W) of the reaction unit produced by the method of producing a reaction unit according to claim 1 or 2, and performing a binding reaction between the capturing antibody (30) and the detection target substance;

(ii) bringing the detection target substance bound to the capturing antibody (30) into contact with a nanoparticle (80) to which a detection antibody (81) having a specific binding property with respect to the detection target substance is fixed, and performing a binding reaction between the detection target substance and the detection antibody (81);

(iii) separating the first substrate (10) and the second substrate (20) after (ii); and

(iv) counting the nanoparticle (80) captured in the separated second substrate (20).

**Patentansprüche**

1.    Verfahren zur Herstellung einer Reaktionseinheit, die Folgendes aufweist:

(a1) Vorbereiten eines ersten Substrates (10), welches ein Substrat mit einer ersten Oberfläche und einer zweiten Oberfläche ist, und von denen zumindest die erste Oberfläche aus Polypropylen zusammengesetzt ist, und die ein oder mehrere Durchgangslöcher (11) hat, welche von der ersten Oberfläche zur zweiten Oberfläche hindurchlaufen, und ein zweites Substrat (20), welches ein Substrat mit einer ersten Oberfläche und einer zweiten Oberfläche ist, und von welchen zumindest die erste Oberfläche aus zumindest einem zusammengesetzt ist, das ausgewählt ist aus der Gruppe bestehend aus Cycloolefin-Polymeren und Cycloolefin-Copolymeren, und die eine Bahnregion (21) hat, in welche konkave Teile und konvexe Teile abwechselnd auf der ersten Oberfläche geformt sind;

(b1) Aufbringen einer fotoaushärtbaren bzw. lichtaushärtbaren Zusammensetzung (41) um eine Öffnung des Durchgangsloches (11) auf der ersten Oberfläche des Substrates (10);

(c1) Emittieren von Licht zu der lichtaushärbaren Zusammensetzung (41), die um die Öffnung des Durchgangslochs (11) herum aufgebracht ist, um eine ausgehärtete Acrylkunststoffschicht (42) zu formen, in welcher die lichtaushärtbare Zusammensetzung ausgehärtet ist, wobei die ausgehärtete Acrylkunststoffschicht (42) Elastizität aufweist; und

(d1) Formen einer Vertiefung (W), welche die Bahnregion (21) als eine Bodenfläche hat und das Durchgangsloch (11) als eine Seitenfläche hat, in dem das erste Substrat (10) und das zweite Substrat (20) unter Verwendung eines Fixierungswerkzeugs (70) fixiert werden, wobei dies eine Vertiefung ist, die geformt wird, indem die ausgehärtete Acrylkunststoffschicht (42), die an der ersten Oberfläche des ersten Substrates (10) geformt ist, mit der ersten Oberfläche des zweiten Substrates (20) nach (c1) in engen Kontakt gebracht wird.

2.  Verfahren zur Herstellung einer Reaktionseinheit, welches Folgendes aufweist:

    (a2) Vorbereiten eines ersten Substrates (10), welches ein Substrat mit einer ersten Oberfläche und einer zweiten Oberfläche ist, und von denen zumindest die erste Oberfläche aus Polypropylen zusammengesetzt ist, und die ein oder mehrere Durchgangslöcher (11) hat, welche von der ersten Oberfläche zur zweiten Oberfläche hindurchlaufen, und ein zweites Substrat (20), welches ein Substrat mit einer ersten Oberfläche und einer zweiten Oberfläche ist, und von welchen zumindest die erste Oberfläche aus zumindest einem zusammengesetzt ist, das ausgewählt ist aus der Gruppe bestehend aus Cycloolefin-Polymeren und Cycloolefin-Copolymeren, und die eine Bahnregion (21) hat, in welche konkave Teile und konvexe Teile abwechselnd auf der ersten Oberfläche geformt sind;

(b2) Aufbringen einer fotoaushärtbaren bzw. lichtaushärtbaren Zusammensetzung (41) um eine Öffnung des Durchgangsloches (11) auf der ersten Oberfläche (10) des Substrates;

(c2) Emittieren von Licht zu der lichtaushärtbaren Zusammensetzung (41), die um die Öffnung in dem Durchgangsloch (11) herum aufgebracht ist, um eine halbausgehärtete Acrylkunststoffschicht (43) zu formen, in welcher die lichtaushärtbare Zusammensetzung halbausgehärtet ist, wobei die halbausgehärtete Acrylkunststoffschicht (43) in einem Zustand ist, in welchem einige polymerisierbare Gruppen in der lichtaushärtbaren Zusammensetzung (41) reagiert haben und einige polymerisierbare Gruppen nicht reagiert haben;

(d2) Formen einer Vertiefung (W), welche die Bahnregion (21) als eine Bodenfläche hat und das Durchgangsloch (11) als eine Seitenfläche hat, welche eine Vertiefung ist, die geformt wird, indem die halbausgehärtete Acrylkunststoffschicht (43), die an der ersten Oberfläche des ersten Substrates (10) geformt ist, mit der ersten Oberfläche des zweiten Substrates (20) nach (c2) in engen Kontakt gebracht wird; und

(e2) Emittieren von Licht zu der halbausgehärteten Acrylkunststoffschicht (43), um eine ausgehärtete Acrylkunststoffschicht (42) zu formen, und dadurch die erste Oberfläche des ersten Substrates (10) und die erste Oberfläche des zweiten Substrates (20) nach (d2) miteinander zu verbinden.

3.  Bausatz zur Erzeugung einer Reaktionseinheit, der Folgendes aufweist:

    ein erstes Substrat (10), welches ein Substrat mit einer ersten Oberfläche und einer zweiten Oberfläche ist, und von denen die erste Oberfläche aus Polypropylen zusammengesetzt ist, und welches ein oder mehrere Durchgangslöcher (11) hat, welche von der ersten Oberfläche zur zweiten Oberfläche hindurchlaufen, und wobei eine ausgehärtete Acrylkunststoffschicht (42), die durch Fotoaushärten bzw. Lichtaushärten einer lichtaushärtbaren Zusammensetzung geformt wird, um eine Öffnung des Durchgangsloches (11) auf der ersten Oberfläche geformt wird; und

    ein zweites Substrat (20), welches ein Substrat mit einer ersten Oberfläche und einer zweiten Oberfläche ist, und von denen zumindest die erste Oberfläche aus zumindest einem zusammengesetzt ist, das ausgewählt ist aus der Gruppe bestehend aus Cycloolefin Polymeren und Cycloolefin Copolymeren, und welches eine Bahnregion (21) hat, in welcher konkave Teile und konvexe Teile abwechselnd auf der ersten Oberfläche geformt sind,

ein Fixierungswerkzeug (70), das konfigurier ist, um das erste Substrat (10) und das zweite Substrat (20) zusammenzudrücken und zu fixieren, wobei eine Vertiefung (W) mit der Bahnregion (21) als Bodenfläche und mit dem Durchgangsloch (11) als Seitenfläche geformt wird, wenn das erste Substrat (10) und das zweite Substrat (20) so fixiert sind, dass die ausgehärtete Acrylkunststoffschicht (42), die an der ersten Oberfläche des ersten Substrates (10) geformt ist, durch das Fixierungswerkzeug (70) in engem Kontakt mit der ersten Oberfläche des zweiten Substrates (20) ist.

4. Bausatz zum Erzeugen einer Reaktionseinheit, der Folgendes aufweist:

ein erstes Substrat (10), welches ein Substrat mit einer ersten Oberfläche und einer zweiten Oberfläche ist, und von denen zumindest die erste Oberfläche aus Polypropylen zusammengesetzt ist, und welches ein oder mehrere Durchgangslöcher (11) hat, die von der ersten Oberfläche zur zweiten Oberfläche hindurchlaufen, und wobei eine halbausgehärtete Acrylkunststoffschicht (43), die durch halbes Fotoaushärten bzw. Lichtaushärten einer lichtaushärtbaren Zusammensetzung erhalten wird, um eine Öffnung des Durchgangsloches (11) an der ersten Oberfläche geformt wird, wobei die halbausgehärtete Acrylkunststoffschicht (43) in einem Zustand ist, in welchem einige polymerisierbare Gruppen in der lichtaushärtbaren Zusammensetzung (41) reagiert haben, und einige polymerisierbare Gruppen nicht reagiert haben; und

ein zweites Substrat (20), welches ein Substrat mit einer ersten Oberfläche und einer zweiten Oberfläche ist, und von denen zumindest die erste Oberfläche aus mindestens einem zusammengesetzt ist, das ausgewählt ist aus der Gruppe bestehend aus Cycloolefin-Polymeren und Cycloolefin-Copolymeren, und welches eine Bahnregion (21) hat, in der konkave Teile und konvexe Teile abwechselnd auf der ersten Oberfläche geformt sind,

wobei eine Vertiefung (W) mit der Bahnregion (21) als eine Bodenfläche und mit dem Durchgangsloch (11) als eine Seitenfläche geformt wird, wenn das erste Substrat (10) und das zweite Substrat (20) verbunden werden, sodass die halbausgehärtete Acrylkunststoffschicht (43), die an der ersten Oberfläche des ersten Substrats (10) geformt ist, in adhäsiver Weise mit der ersten Oberfläche des zweiten Substrates (20) verbunden wird.

5. Verfahren zum Messen einer Detektionszielsubstanz, welches Folgendes aufweist:

(i) Einbringen einer Probe, welche die Detektionszielsubstanz enthält, mit einer spezifischen Bindungseigenschaft bezüglich eines aufnehmenden Antikörpers (30) in eine Vertiefung (W), in welcher der aufnehmende Antikörper (30) in einer Bahnregion (21) fixiert ist, welche die Vertiefung (W) der Reaktionseinheit ist, die durch das Verfahren zur Erzeugung einer Reaktionseinheit nach Anspruch 1 oder 2 erzeugt wurde, und Ausführen einer Bindungsreaktion zwischen dem aufnehmenden Antikörper (30) und der Detektionszielsubstanz;

(ii) In-Kontakt-Bringen der Detektionszielsubstanz, die an den aufnehmenden Antikörper (30) gebunden ist, mit einem Nanopartikel (80), an dem ein Detektionsantikörper (81) mit einer spezifischen Bindungseigenschaft bezüglich der Detektionszielsubstanz fixiert ist, und Ausführen einer Bindungsreaktion zwischen der Detektionszielsubstanz und dem Detektionsantikörper (81);

(iii) Trennen des ersten Substrates (10) und des zweiten Substrates (20) nach (ii); und

(iv) Zählen der Nanopartikel (80), die in dem abgetrennten zweiten Substrat (20) aufgenommen sind.

## Revendications

1. Procédé de production d'une unité de réaction, comprenant :

(a1) préparer un premier substrat (10) qui est un substrat ayant une première surface et une seconde surface et dont au moins la première surface est composée de polypropylène, et qui a un ou plusieurs trous traversants (11) qui pénètrent de la première surface à la seconde surface, et un second substrat (20) qui est un substrat ayant une première surface et une seconde surface et dont au moins la première surface est composée d'au moins un élément choisi dans le groupe constitué de polymères de cyclooléfine et de copolymères de cyclooléfine, et qui a une région de piste (21) dans laquelle des parties concaves et des parties convexes sont formées en alternance sur la première surface ;

(b1) appliquer une composition photodurcissable (41) autour d'une ouverture du trou traversant (11) sur la première surface du premier substrat (10)

(c1) émettre de la lumière vers la composition photodurcissable (41) appliquée autour de l'ouverture du trou traversant (11) pour former une couche de résine acrylique durcie (42) dans laquelle la composition photodurcissable est durcie, la couche de résine acrylique durcie (42) ayant une élasticité ; et

(d1) former une cavité (W) ayant la région de piste (21) en tant que surface inférieure et le trou traversant (11) en tant que surface latérale en fixant le premier substrat (10) et le second substrat (20) en utilisant un outil de fixation (70), qui est une cavité formée en amenant la couche de résine acrylique durcie (42) formée sur la première surface du premier substrat (10) en contact étroit avec la première surface du second substrat (20) après (c1).

2. Procédé de production d'une unité de réaction, comprenant :

(a2) préparer un premier substrat (10) qui est un substrat ayant une première surface et une seconde surface et dont au moins la première surface est composée de polypropylène, et qui a un ou plusieurs trous traversants (11) qui pénètrent de la première surface à la seconde surface, et un second substrat (20) qui est un substrat ayant une première surface et une seconde surface et dont au moins la première surface est composée d'au moins un élément choisi dans le groupe constitué de polymères de cyclooléfine et de copolymères de cyclooléfine, et qui a une région de piste (21) dans laquelle des parties concaves et des parties convexes sont formées en alternance sur la première surface ;

(b2) appliquer une composition photodurcissable (41) autour d'une ouverture du trou traversant (11) sur la première surface (10) du premier substrat

(c2) émettre de la lumière vers la composition photodurcissable (41) appliquée autour de l'ouverture du trou traversant (11) pour former une couche de résine acrylique semi-durcie (43) dans laquelle la composition photodurcissable est semi-durcie, la couche de résine acrylique semi-durcie (43) est dans un état dans lequel certains groupes polymérisables dans la composition photodurcissable (41) ont réagi et certains groupes polymérisables n'ont pas réagi ;

(d2) former une cavité (W) ayant la région de piste (21) en tant que surface inférieure et le trou traversant (11) en tant que surface latérale, qui est une cavité formée en amenant la couche de résine acrylique semi-durcie (43) formée sur la première surface du premier substrat (10) en contact étroit avec la première surface du second substrat (20) après (c2) ; et

(e2) émettre de la lumière vers la couche de résine acrylique semi-durcie (43) pour former une couche de résine acrylique durcie (42) et ainsi lier la première surface du premier substrat (10) et la première surface du second substrat (20) après (d2).

3. Kit pour produire une unité de réaction, comprenant :

un premier substrat (10) qui est un substrat ayant une première surface et une seconde surface et dont au moins la première surface est composée de polypropylène, et qui a un ou plusieurs trous traversants (11) qui pénètrent de la première surface à la seconde surface, et dans lequel une couche de résine acrylique durcie (42) formée par photodurcissement d'une composition photodurcissable est formée autour d'une ouverture du trou traversant (11) sur la première surface ; et

un second substrat (20) qui est un substrat ayant une première surface et une seconde surface et dont au moins la première surface est composée d'au moins un élément choisi dans le groupe constitué de polymères de cyclooléfine et de copolymères de cyclooléfine, et qui a une région de piste (21) dans laquelle des parties concaves et des parties convexes sont formées en alternance sur la première surface,

un outil de fixation (70) configuré pour presser et fixer le premier substrat (10) et le second substrat (20),

dans lequel une cavité (W) ayant la région de piste (21) en tant que surface inférieure et le trou traversant (11) en tant que surface latérale est formée lorsque le premier substrat (10) et le second substrat (20) sont fixés de sorte que la couche de résine acrylique durcie (42) formée sur la première surface du premier substrat (10) est en contact étroit avec la première surface du second substrat (20) par l'outil de fixation (70).

4. Kit pour produire une unité de réaction, comprenant :

un premier substrat (10) qui est un substrat ayant une première surface et une seconde surface et dont au moins la première surface est composée de polypropylène, et a un ou plusieurs trous traversants (11) qui pénètrent de la première surface à la seconde surface et dans lequel une couche de résine acrylique semi-durcie (43) obtenue par photosemi-durcissement d'une composition photodurcissable est formée autour d'une ouverture du trou traversant (11) sur la première surface, la couche de résine acrylique semi-durcie (43) est dans un état dans lequel certains groupes polymérisables dans la composition photodurcissable (41) ont réagi et certains groupes polymérisables n'ont pas réagi ; et

un second substrat (20) qui est un substrat ayant une première surface et une seconde surface et dont au moins la première surface est composée d'au moins un élément choisi dans le groupe constitué de polymères de cyclooléfine et de copolymères de cyclooléfine, et qui a une région de piste (21) dans laquelle des parties concaves et des parties convexes sont formées en alternance sur la première surface,

dans lequel une cavité (W) ayant la région de piste (21) en tant que surface inférieure et le trou traversant (11) en tant que surface latérale est formée lorsque le premier substrat (10) et le second substrat (20) sont liés de sorte que la couche de résine acrylique semi-durcie (43) formée sur la première surface du premier substrat (10) est liée de manière adhésive à la première surface du second substrat (20).

5. Procédé de mesure d'une substance cible de détection, comprenant :

   (i) ajouter un échantillon contenant une substance cible de détection ayant une propriété de liaison spécifique par rapport à un anticorps de capture (30) à une cavité (W) dans laquelle l'anticorps de capture (30) est fixé dans une région de piste (21), qui est la cavité (W) de l'unité de réaction produite par le procédé de production d'une unité de réaction selon la revendication 1 ou 2, et réaliser une réaction de liaison entre l'anticorps de capture (30) et la substance cible de détection ;
   (ii) amener la substance cible de détection liée à l'anticorps de capture (30) en contact avec une nanoparticule (80) à laquelle un anticorps de détection (81) ayant une propriété de liaison spécifique par rapport à la substance cible de détection est fixé, et réaliser une réaction de liaison entre la substance cible de détection et l'anticorps de détection (81) ;
   (iii) séparer le premier substrat (10) et le second substrat (20) après (ii) ; et
   (iv) compter la nanoparticule (80) capturée dans le second substrat séparé (20).

## FIG. 1

## FIG. 2A

## FIG. 2B

## FIG. 3

## FIG. 4A

## FIG. 4B

*FIG. 5*

## FIG. 6A

## FIG. 6B

## FIG. 7A

## FIG. 7B

## FIG. 8A

## FIG. 8B

## FIG. 8C

FIG. 9A

FIG. 9B

FIG. 10

## FIG. 11A

## FIG. 11B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021082279 A
- JP 2017207289 A
- US 2020217864 A
- US 2016089649 A
- US 2008063572 A